# EUROPEAN PATENT APPLICATION

(11) **EP 4 282 369 A1**
(43) Date of publication of application: **29.11.2023**
(21) Application number: 22175438.5
(22) Date of filing: 25.05.2022
(51) Int. Cl.: A61B 90/00, A61M 25/04, A61M 25/10, G01N 29/36

(54) **A METHOD AND CATHETER CONTROL ASSEMBLEY FOR DETERMINING A FUNCTIONAL PARAMETER OF A CATHETER**

(71) Applicant: Wellspect AB, 431 21 Mölndal (SE)
(72) Inventor: D'Espindula, Gabriel, 418 71 Göteborg (SE); Chandrasekaran, Adarsh, 431 21 Mölndal (SE)
(74) Representative: Lind Edlund Kenamets Intellectual Property AB

(57) **Abstract**

A method and catheter control assembly determines a functional parameter of a catheter. Th catheter is connected to a catheter connector interface, wherein the catheter connector interface comprises a first lumen configured to be in fluid communication with an inflatable retention element of the catheter and a second lumen configured to be in fluid communication with a main lumen of the catheter. A sound wave, such as an acoustic pulse or a sound impulse, is input into the first lumen, e.g. by a speaker, and at least one parameter of a reflection of the sound wave in the first lumen is measured, e.g. with a microphone. Based on the measured parameter, a functional parameter of the catheter, such as whether the catheter has been previously used or not, the size of the catheter, etc.

## Description

### TECHNICAL FIELD OF THE INVENTION

The present invention relates to a method and a catheter control assembly for determining a functional parameter of a connected catheter.

### BACKGROUND OF THE INVENTION

A catheter is a medical instrument useable for many types of uses and applications, comprising an elongated body through which one or more channels extends. By inserting a catheter in a bodily cavity or through the skin of a patient the channels of the catheter may be used to perform a wide range of medical procedures including enabling insertion of surgical instruments, drainage of fluids or administration of fluids through the one or more channels extending through the elongated body. The number and type of channels available in a catheter depends on the particular type of catheter and its intended use.

For instance, catheters may be used to perform rectal irrigation, such as trans anal irrigation (TAI), which involves introducing a catheter into the anal canal or rectum of a patient and injecting a fluid (e.g. water) to flush out stool and stimulate the bowels of the patient. Rectal catheters used for this purpose are often provided with a retention element, which may function to retain the catheter in an inserted position, and/or to form a seal against the anal canal when inserted into the rectum of a user. The retention element may e.g. be in the form of a cone which surrounds the elongated body and seals the anal canal or rectum when the catheter is used to inject fluid. Alternatively, a rectal catheter may be provided with an inflatable element (e.g. a balloon element) which surrounds the elongated body, and which is inflated inside the anal canal or rectum to create a seal and/or to retain the catheter in place. To this end, rectal catheters may feature at least two separate channels, a first channel for the injection of irrigation fluids and a second channel which at a first end is terminated inside the inflatable element for inflation of the inflatable element. By injecting a fluid, such as air or water, in the channel of the inflatable element the level of inflation may be controlled such that the inflatable element creates a seal and/or is inflated to allow the catheter to be retained in the inserted position. When the catheter is to be removed, the same channel may be used for deflation of the inflatable element.

A problem with prior solutions is that it could be difficult for medical professionals to keep track of the different types of catheters and also to separate used catheters from new catheters. There is also a risk that, by accident, a catheter is used and operated in an erroneous way, such as a catheter prescribed for children being inflated in the same way as a catheter for adults. Such erroneous use could potentially be dangerous for the user. Commonly a catheter is primed some time before use wherein the priming procedure may involve connecting the catheter to a catheter junction and e.g. flushing a fluid through one or more of the channels. In light of these problems, a solution has been proposed in WO20142185 involving attaching one or more magnets on the connector base of the catheter and using a Hall Effect sensor coupled to a controller in the catheter junction to detect the number or type of magnets on the catheter connector. By equipping different types of catheters with different numbers of magnets the controller of the catheter junction may determine the type of catheter based on measurements from the Hall Effect sensor and the controller may e.g. ensure that a proper procedure will be performed with the connected catheter or that correct information will be displayed for the medical professional.

A problem with this solution is however that existing catheters then need to be provided with a correct number of magnets to allow the Hall Effect sensor of the catheter junction to properly detect the type of catheter and that the system is incapable of determining if a catheter has been previously used. This known solution is complicated and costly, and cumbersome to use.

### SUMMARY OF THE INVENTION

In view of the drawbacks of existing solutions there is a need for an improved method and catheter arrangement capable of determining the type of catheter. It is a purpose of the present invention to provide such an improved method and catheter arrangement which facilitates determination of a functional parameter of a catheter.

According to a first aspect of the invention there is provided a method for determining a functional parameter of a catheter, the method comprising:
a) connecting said catheter to a catheter connector interface, said catheter connector interface comprising a first lumen configured to be in fluid communication with an inflatable retention element of the catheter and a second lumen configured to be in fluid communication with a main lumen of the catheter;
b) inputting a sound wave into the first lumen;
c) measuring at least one parameter of a reflection of the sound wave in the first lumen; and
d) determining, based on the measured parameter, a functional parameter of the catheter.

The inflatable retention element can be inflated with any type of fluid. By fluid is meant any liquid, gas or material which flows (such as a foam) or combination thereof. In embodiments, the fluid is a gas, such as ambient air, or a liquid, such as an irrigation liquid, e.g. water or saline.

By functional parameter is meant a parameter indicating a property of the catheter. The property may be one or more properties selected from a group comprising: (a) whether the catheter comprises an inflatable retention element, (b) whether the inflatable retention element of the catheter has been used previously, i.e. whether the inflatable retention element has been inflated, and preferably fully inflated, previously, (c) whether the inflatable retention element of the catheter is new, i.e. that the inflatable retention element has not been inflated previously, or at least not fully inflated, and (d) the size/volume of the inflatable retention element. Determining the size/volume of the inflatable retention element may comprise determining the absolute size/volume of the inflatable retention element or determining if the inflatable retention element is of a first size or second size. Determining the size/volume of the inflatable retention element may comprise determining which size/volume out of at least two sizes/volumes that corresponds to the inflatable retention element. The catheter may be any type of catheter, preferably the catheter is a rectal irrigation catheter, a urinary tract catheter or a ureteric balloon catheter.

The inflatable retention element may be a balloon element made from an elastic or resilient material.

By sound and sound wave is here meant any wave that behaves and propagates like an acoustic wave. The sound wave preferably has a frequency in the audible frequency range, such as in the range from 20 Hz to 20 kHz. However, frequencies above the audible frequency range, i.e. ultrasound, may also be used, such as frequencies greater than 20 kHz, such as up to 100 kHz, 500 kHz, or more. Frequencies below the audible frequency range, i.e. infrasound, may also be used, such as frequencies below 20 Hz, such as down to 1 Hz, 0.1 Hz or less.

By a reflection of the sound wave is here meant any sound waves resulting from a reflection of the initial sound wave, due to reflections from the walls of the first lumen, from the inflatable retention element, etc. These reflected sound waves may be referred to as reverberation. The reverberation may e.g. be determined in accordance with the standard ISO 3382-1-2009. For example, a reverberation time may be determined to be the duration of a sound energy density to decrease by 60 dB after the source emission has stopped. The reflected sound waves may also be referred to as an echo. Generally, echo may be used for reflections providing distinguishable repetitions of the initial sound, whereas reverberation may be used for reflections providing a more uniformly, non-distinct reflected sound pattern.

In a preferred embodiment, the sound wave has a frequency within the audible frequency range, and preferably within the range of 20 Hz - 20 kHz, and more preferably in the range of 1-10 kHz. For such embodiments, relatively low-cost and simple microphones may be used as sensors to detect parameters related to the input sound wave and/or the reflected sound wave.

The present invention is at least partially based on the understanding that the reflected sound wave, i.e. the echo, depends on the functional parameter of the catheter. Therefore, measuring of one or more parameters related to the reflected sound wave can be used to determine various functional parameters related to the catheter, and in particular related to the inflatable retention element. Therefore, by inputting a sound wave, e.g. in the form of an acoustic pulse, or sound impulse, and measuring one or more parameters of the reflected sound wave in the first lumen, the measured parameter(s) will yield the functional parameter(s) in a very simple, quick, reliable and cost-effective way, without necessitating e.g. a new type of catheter or retrofitting existing catheters with e.g. magnets that can be detected with Hall Effect sensors.

In an embodiment, the catheter control assembly is arranged to determine whether a catheter connected to the assembly comprises an inflatable retention element or not. In an embodiment, the catheter control assembly is arranged to determine whether the inflatable element of a catheter connected to the assembly has been previously inflated or not. In an embodiment, the catheter control assembly is arranged to determine a size or particular type of a catheter connected to the assembly.

It is of advantage to be able to ensure that medical professionals, or patients performing self-treatment, use a catheter that is intended for the treatment which is to be performed. Hereby, it may be ensured that the catheter control assembly is not used with a catheter for which it is not suited, or that the catheter control assembly is controlled in a way unsuitable for the catheter. Additionally, or alternatively, for single use catheters it may be of advantage that medical professionals, or patients performing self-treatment, do not use a catheter that has been used previously. A used catheter may have properties that have been impaired by the previous use, may be contaminated, or may for other reasons be unsuitable for a repeated, subsequent use.

With the method of the present invention the functional parameter of a catheter may be automatically determined when the catheter is connected to the catheter connector interface, e.g. during a priming of the catheter. The functional parameter may then be presented to the medical professional, or patient performing self-treatment, so as to e.g. issue a warning if a previously used catheter has been connected to the catheter connector interface. Additionally or alternatively, the functional parameter may be used to control the treatment procedure, e.g. if the functional parameter indicates that the catheter does not comprise an inflatable retention element a valve or pressure source may be controlled to ensure that no fluid is injected to the first lumen during the treatment procedure.

The measuring of the at least one parameter of the reflection of the sound wave in the first lumen preferably comprises measuring of at least one of, and more preferably at least two of, and most preferably all three of: The time it takes for the reflection of the sound wave to be detectable after inputting of the sound wave into the first lumen; the frequency or frequencies of the reflected sound wave; the phase of the reflected sound wave; and the amplitude of the reflected sound wave.

It has been found that the time it takes for the reflection of the sound wave to be detectable after inputting of the sound wave into the first lumen may be used as an indication of the path length of the first lumen, the size of an internal cavity of the inflatable retention element, the presence of obstacles in the first lumen, etc.

It has further been found that the frequency or frequencies of the reflected sound wave may be indicative of e.g. the status and condition of the inflatable retention element, such as whether it has previously been fully inflated or not, but also indicative of other conditions, such as the size and shape of an internal cavity of the inflatable retention element, etc. In particular, the measured frequency or frequencies of the reflected sound wave may be compared to the frequency or frequencies of the input sound wave. To this end, the difference in frequency, in absolute or relative terms, may be used as an enhanced parameter, based on the measured frequency, to determine the one or more functional parameter. For example, the input sound wave may have a frequency of 5.0 kHz, and the reflected sound wave may be measured to be 5.2 kHz. In this example, the controller may directly use the frequency value, i.e. 5.2 kHz, as the measured parameter for determination of the one or more functional parameter(s). In particular this is useful when the input sound wave always has the same frequency, such as 5.0 kHz. Alternatively, the controller may calculate the absolute difference between the measured frequency and the input frequency, such as +0.2 kHz, and use this value for the determination of the one or more functional parameter(s). Alternatively, the controller may calculate the relative difference between the measured frequency and the input frequency, such as +4%, and use this value for the determination of the one or more functional parameter(s). The frequency of the input sound wave may be predetermined, and consequently be known to the controller. Alternatively, the controller may be arranged to measure the frequency or frequencies also of the input sound wave. The absolute and relative frequency differences may be particularly useful when the frequency or frequencies of the input sound wave varies, intentionally or unintentionally.

It has further been found that the amplitude of the reflected sound wave may be used to determine the functional parameter(s). In case the sound wave comprises more than one frequency, the amplitude for each frequency may further be considered separately. Thus, the amplitude may be considered in the frequency domain, as separate amplitudes in the frequency spectrum. The amplitude may be indicative of the absorption of the sound that occurs within the first lumen and in the inflatable retention member. The absorption may e.g. be dependent on the elasticity of the wall material of the inflatable retention member, which in turn may be dependent on whether the inflatable retention member has been used and inflated previously, and also of how many times inflation has occurred. The amplitude may also be dependent on e.g. the length of the first lumen. In particular, the measured amplitude of the reflected sound wave may be compared to the amplitude of the input sound wave. To this end, the difference in amplitude, in absolute or relative terms, may be used as an enhanced parameter, based on the measured amplitude, to determine the one or more functional parameter. For example, the input sound wave may have an amplitude of 10 dB, and the reflected sound wave may be measured to have an amplitude of 1 dB. In this example, the controller may directly use the measured amplitude value, i.e. 1 dB, as the measured parameter for determination of the one or more functional parameter(s). In particular this is useful when the input sound wave always has the amplitude, such as 10 dB. Alternatively, the controller may calculate the absolute difference between the measured amplitude and the input amplitude, such as -9 dB, and use this value for the determination of the one or more functional parameter(s). Alternatively, the controller may calculate the relative difference between the measured amplitude and the input amplitude, such as -65%, and use this value for the determination of the one or more functional parameter(s). The amplitude of the input sound wave may be predetermined, and consequently be known to the controller. Alternatively, the controller may be arranged to measure the amplitude also of the input sound wave. The absolute and relative amplitude differences may be particularly useful when the amplitude of the input sound wave varies, intentionally or unintentionally.

The phase may be measured and considered in a similar way as discussed in the foregoing in relation to frequency and amplitude, and can also be used, additionally or alternatively, to determine one or more functional parameters.

The measured parameters make it possible to directly or indirectly determine the system's impulse response. The input signal may, from a mathematical point of view, be seen as a set of scaled and shifted unit impulses. Thus, the input signal can be decomposed into a set of impulses. The output signal, which is the one measured by the sensor, may be seen as a convolution of the input signal, where the convolution depends on the system's impulse response. The impulse response is, as discussed in the foregoing, dependent on the shape and size of the internal cavity formed by the first lumen and the balloon, but also on other factors, such as properties of the material forming the internal cavity, surrounding environment, outside the cavity, etc. Mathematically, this may be expressed as x[n] * h[n] = y[n], where x[n] is the input signal, h[n] is the impulse response, y[n] is the output signal, and * is the convolution. Determination of any one of the impulse response, input signal and output signal, based on the other two, are possible by digital signal processing, as is per se known in the art.

The sound wave input into the first lumen may preferably be in the form of a sound wave having a predetermined limited duration, and preferably in the form of a sound impulse. A sound impulse may be seen as one or more, such as a set of, scaled and shifted unit impulses. Such sound impulses/pulses may be very distinct, and easy to measure on. The predetermined limited duration may be between 1 millisecond and 1 second and preferably between 1 millisecond and 0.5 seconds and more preferably between 1-10 milliseconds. The sound impulse may be a pulse that is shorter, or much shorter, than the time response of the system.

However, shorter impulses/pulses may also be used, such as in the range of 0.01-1 milliseconds, and preferably in the range of 0.1-0.5 milliseconds. Such very short impulses/pulses may be useful since the input impulse/pulse will not be present and measured at the sensor, such as the microphone, when the returned sound wave is measured. The speed of sound is about 343 m/s in normal air, and consequently the sound propagates about 34 cm over 1 millisecond. The duration of the impulse/pulse may consequently, in one embodiment, be set to a value lower than the time it would take for the sound to travel back and forth in the internal lumen.

The sound impulse preferably has a relatively large initial energy, providing an initial transient peak in amplitude, and a relatively short duration, similar to the sound of a pistol shot, a clap or the like.

The functional parameter to be determined may be at least one of: the size of the inflatable retention element, the status or condition of the inflatable retention element, and the size of the catheter. Additionally, or alternatively, the functional parameter may be at least one of: the presence of a catheter and the presence of a fluid communication with the inflatable retention element. Preferably, more than one of these functional parameters may be determined, and most preferably all of them. In an embodiment, the functional parameter is indicative of at least one of: whether the catheter comprises an inflatable retention element, whether the inflatable retention element is new or used, and a size of the inflatable retention element.

The measurement and determination of the functional parameter may be performed only upon request from a user. However, preferably it is made automatically. The automatic activation may occur e.g. upon the happening of certain events, such as when the control unit is powered and activated, when the pump is operated, when a catheter is connected, etc. The measurement and determination may also be repeated continuously or regularly, for example during the entire duration of a catheterization or irrigation process, at all time when the catheter is connected, at all time when the pump is active, etc.

When a sound wave is injected/input into the first lumen the reflection of the sound wave will depends on the functional parameter of the catheter. By measuring the reflected sound wave, and in particular the propagation time, the frequency and/or the amplitude of the reflected sound wave, the functional parameter of the catheter may be determined based on the measurements. The measurements can e.g. be made by a conventional microphone.

In some implementations, the measured parameters of the reflected sound wave may be used to determine that the catheter comprises a new inflatable retention element, or alternatively that the catheter comprises a used inflatable retention element. A catheter comprising an inflatable retention element which has not been used previously provides a different response in respect of frequency, amplitude and possibly also in duration, than a previously used catheter. Degradation occurring from multiple uses may also be determinable in the same way. Additionally, the determined functional parameter may indicate that the catheter is not provided with an inflatable retention element. A catheter without an inflatable retention element would provide a shorter duration for the sound wave to be reflected back, and would possibly provide a different response in terms of frequency and amplitude. In particular, the difference in measured parameters will be highly significant when the catheter has a sealed inflation lumen, or no inflation lumen at all, meaning that the first lumen of the catheter connector interface is not in fluid communication with any volume in the catheter. Thus, the measured parameters may be used to separate such catheters from catheters with any form of inflatable retention element.

The determination of the one or more functional parameter of the catheter, based on the measured one or more parameters of the reflection of the sound wave in the first lumen, can be made by an artificial neural network. The neural network may be a feedforward network or a recurrent network. The neural network may be trained by measuring the one or more parameters of the reflected sound wave for a plurality of catheters having known functional parameters.

The situation may be seen as a linear time variant system, where the output dies not depend on when the input sound was applied, and where the input-output characteristics do not change with time.

The neural network is preferably first trained with selected samples, and subsequently the trained network may be used to detect the functional parameters of the catheter.

The measured reflection of the sound wave may be represented as a mel-frequency cepstrum (MFC) representation. This may be a representation of the short-term power spectrum of a sound based on a linear cosine transform of a low power spectrum of a nonlinear mel scale of frequency. The MFC can be computed by mel-frequency cepstral coefficients (MFCCs), collectively providing the MFC. They are derived from a type of cepstral representation of the audio clip (a nonlinear "spectrum-of-a-spectrum"). The difference between the cepstrum and the mel-frequency cepstrum is that in the MFC, the frequency bands are equally spaced on the mel scale, which approximates the human auditory system's response more closely than the linearly-spaced frequency bands used in the normal spectrum.

The MFC can e.g. be obtained by Mel-Spectograms, available through MatLab. Mel-Spectograms, or similar image representations of sound, may show a representation of the sound as a frequency, e.g. on the Y-axis, over time, e.g. on the X-axis. In addition, the amplitude or power may be shown by the use of different colors in the diagram.

The neural network may e.g. be a pre-trained neural network, such as known neural networks used for images, pre-trained neural networks for audio, etc. The Mel-Spectogram or other images representing the sound may then be used as input images. The trained neural networks may e.g. be ImageNet, YAMnet, or GoogLeNet, which are all commercially available. If e.g. YAMNet is used, as available through MatLab, the final layers, such as the fully connected layer (dense), the softmax layer (softmax) and the classification layer (sound) may be replaced in accordance with the contextual circumstances of the sound in the catheter, in order for it to learn from the new samples of data.

Further, to make the process more efficient, the network may be divided into two or more sub-networks, instead of using one network for classifying all the different configurations. For example, four different training networks may be used. The sub-networks may e.g. be related to whether the catheter has been used before, the catheter size, the number of previous inflations and the degree of inflation.

Alternatively, one or more of the functional parameters may be determined by comparing a measured parameter for the reflected sound wave with a database of previously measured parameter samples obtained for catheters with different functional parameters and determining the closest matching parameter samples. The associated functional parameter of the closest matching sequence may be the determined functional parameter. It is noted that many alternatives exist for determining the functional parameter based on parameter(s) of the reflected sound wave given reference measurements of the parameter for characters with known functional parameters.

According to a second aspect of the invention there is provided a catheter control assembly comprising:
a catheter connector interface, comprising:
   a first lumen configured to be in fluid communication with an inflatable retention element of the catheter;
   a second lumen configured to be in fluid communication with a main lumen of a catheter;
   a sound generator arranged to input a sound wave into the first lumen; and
a catheter controlling unit, the catheter controlling unit being configured to
   measure at least one parameter of a reflection of the sound wave in the first lumen; and
   determine, based on the measured pressure parameter, a functional parameter of the catheter.

For instance, the catheter controlling unit may comprise a controller and a sensor, such as a microphone, for measuring the at least one parameter of the reflected sound wave. The controller may be used to control the sound generator, such as a speaker, to input the sound wave into the first lumen. The speaker may e.g. be a speaker as is per se known from earphones. However, other sound generators may also be used, and generally, any device able to generate a specific and defined sound may be used as a sound generator, such as a piezo actuator, a pump, a motor, etc.

Thus, in one embodiment, the catheter control assembly further comprises a microphone connected to said catheter controlling unit for measuring of the at least one parameter of the reflected sound wave. For example, the microphone may be a condenser microphone, for example having a range of 20 Hz to 18 kHz.

The catheter control assembly may further comprise a pump, and preferably an electric pump. The pump may be arranged integrated within a single housing of the catheter control assembly. However, alternatively, the pump may be provided as a separate, external pump. The catheter control assembly may further comprise a valve, separating an output of the pump from the catheter connector interface. Additional components may also be provided in the catheter control assembly, such as sensors, an energy source, such as a battery, additional pumps, etc. A reservoir for accommodation of a supply of irrigation liquid may also be provided, arranged integrated or connected to the assembly.

In an embodiment, the pump may also be arranged to generate a specific sound, such as a set of sound impulses/pulses, or a constant sound of one or more frequencies. In such embodiments, the sound from the pump may be used as the input sound for the first lumen, to generate the reflected sound wave.

However, preferably the sound wave input into the first lumen is generated by a dedicated sound generator. Hereby, the sound wave is easier to control, and is generally also more predictable and constant over time. In one embodiment, the sound generator is a speaker.

The controlling unit may be realized in software executed in a processor, such as a CPU, a microcontroller, etc. The controlling unit may also be realized partly or fully in hardware. The controlling unit is preferably arranged as an integrated part of the catheter control assembly, and preferably arranged within a housing thereof. However, alternatively, the controlling unit may be arranged externally, and connected to the rest of the assembly through a wired or wireless communication connection.

According to a third aspect of the invention there is provided a computer program product comprising instructions which, when executed by a computer, performs the method according to the first aspect of the invention. The computer program product, such as computer executable software, may be provided on a data carrying article of manufacture, such as a data storage.

The invention according to the second and third aspect features the same or equivalent benefits as the invention according to the first aspect. Any functions described in relation to a method may have corresponding features in a device or arrangement, and vice versa.

### BRIEF DESCRIPTION OF THE DRAWINGS

The present invention will be described in more detail with reference to the appended drawings, showing currently preferred embodiments of the invention.
Figure 1 depicts a block diagram of a catheter arrangement according to some embodiments of the present invention.
Figure 2 illustrates schematically, in cross-section, a catheter with an inflatable retention element according to some embodiments of the present invention.
Figure 3 illustrates schematically, in cross-section, a catheter arrangement connected to a catheter with an inflatable retention element according to some embodiments of the present invention.
Figure 4 is a flowchart illustrating a method according to some embodiments of the present invention.
Figure 5 is a schematic illustration of a controller unit for use in an embodiment of the present invention

### DETAILED DESCRIPTION OF CURRENTLY PREFERRED EMBODIMENTS

In the following detailed description preferred embodiments of the invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length of the catheter, etc. Further, even though the discussed embodiments are in particular suitable for use in rectal irrigation assemblies, i.e. catheter control assemblies to be connected to rectal catheters, e.g. for use in trans anal irrigation, similar catheter control assemblies may also be used for other applications, such as for dilation catheters, venous catheters, urinary catheters, and the like.

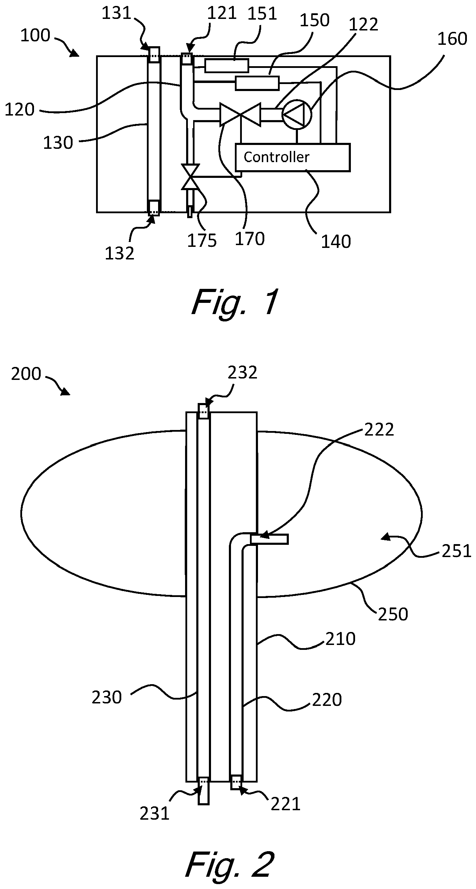

Fig. 1 depicts a block diagram of a catheter control assembly 100 comprising a second (passthrough) lumen 130 and a first lumen 120 wherein the second lumen 130 is configured to be in fluid communication with a main lumen of a catheter via a catheter connection interface through a catheter connection port 131 and the first lumen is configured to be in fluid communication with an inflatable retention element of the catheter via the catheter connection interface through an inflation port 121. The illustrated catheter control assembly 100 comprises two lumens, a first lumen 120 and a second lumen 130, but may comprise additional lumens in any suitable arrangement. For instance, the catheter control assembly 100 may comprise at least two second (passthrough) lumens 130 in addition to the first lumen 120 and the first lumen 120 and second lumen(s) 130 may be arranged concentrically as opposed to the side-by-side arrangement of the embodiment shown in fig. 1.

The second lumen 130 extends between the first catheter connection port 131 and the first passthrough port 132 to enable e.g. a fluid or a surgical instrument to be passed through the second lumen 130 of the catheter control assembly 100. Although the catheter control assembly 100 in fig. 1 is depicted as single unit the catheter control assembly 100 may comprise separate units. For instance, a catheter connector interface comprising the second lumen 130 and the first lumen 120 may be provided as at least one separate unit(s) while the controller 140 and sensor 150 are provided as another separate unit (a catheter controlling unit) and the pressure source 160 is provided as yet another separate unit.

The second lumen 130 may e.g. be connected to a reservoir accommodating a supply of irrigation liquid, such as water, and a manual or electric pump may be provided to pump the irrigation liquid from the reservoir to the second lumen 130.

The catheter control assembly 100 preferably comprises a pressure source 160 in fluid communication with the first lumen 120. The pressure source 160 may be a pressure vessel containing a fluid under pressure and/or a pump configured to pump a fluid into the first lumen 120. The pump may be any suitable pump such as a mechanical pump, electric pump or electromechanical pump. For example, the pump is a positive displacement pump, a rotary positive displacement pump (e.g. a screw pump or gear pump), a reciprocating pump (e.g. a plunger/piston pump or a diaphragm pump) or a peristaltic pump. Preferably, the pump is an electric pump.

Another pressure source or pump may be connected to/passed through the passthrough port 132 to e.g. inject or remove a fluid to the catheter when performing treatment. However, in embodiments, the same pressure source/pump 160 may be used both for inflation of the inflatable retention element through the first lumen 120 and for pumping a liquid through the second lumen 130.

The pressure source 160 may be associated with a valve 170 which separates the pressure source 160 from the first lumen 120. In some implementations, the pressure source 160 is a pressure vessel and the valve 170 is a valve of the pressure vessel, accordingly the valve 170 and a pressure vessel may together form a controllable pressure source. In some implementations, there is a buffer volume 122 between the pressure source 160 and the valve 170. The valve 170 may be three-way valve wherein the valve 170 is further configured to vent the first lumen 120. Alternatively, a venting valve 175 is provided which is configured to vent the first lumen 120 after the predetermined period of time. The venting valve 175 may be configured to be closed during use, when the inflatable retention element is about to be inflated, and when also when the inflatable retention element is in an inflated state. After use, the valve 175 may be operated for deflation of the inflatable retention element. The venting valve 175 may be controlled by the controller 140.

The catheter control assembly 100 may further comprise a sound generator or sound source 150, connected to the first lumen 120 to input a sound wave into the first lumen 120. The sound generator 150 may e.g. be realized as a speaker. The sound generator 150 is further preferably connected to the controller 140, thereby enabling control of the sound generator 150 from the controller.

The catheter control assembly 100 may further comprise a sensor 151 connected to the first lumen 120, and configured to sense a reflected soundwave parameter in the first lumen 120 related to the soundwave generated by the sound generator 150. For instance, the sensor 151 may be realized as a microphone. The sensor 151 is further preferably connected to the controller 140, whereby signals corresponding to the sensed soundwave parameters can be forwarded to the controller for analysis and possible actions.

Moreover, the catheter control assembly 100 may comprise a controller 140 for controlling at least the sound generator 150, and to receive sensor data from the sensor 151. The controller may further be arranged to control the pressure source 160 and/or the valve 170.

The controller 140 may control, and be operatively connected to, the sound generator 150 and the sensor 151 to perform measurements at suitable times. As will be discussed in more detail in the following.

Fig. 2 illustrates schematically a catheter 200 comprising an elongated body 210 through which at least two lumens 220, 230 extend at least partially. The catheter may comprise a catheter connector, connectable to the catheter connector interface of the catheter control assembly. In the depicted embodiment a main lumen 230 extends from a first connector port 231 of a first end of the elongated body 210 to a second port 232 of a second end of the elongated body 210. When in use, the main lumen 230 of the catheter 200 may be used to introduce or remove a surgical instrument or a fluid to/from the patient via the first connector port 231 and the second port 232. The catheter 200 may comprise more than one main lumen 230 extending from substantially the first end to the second end of the catheter 200 such as two, three or more main lumens 230. Multiple main lumens 230 may be provided to enable simultaneous use of an instrument, administering of at least one fluid and/or removal of at least one fluid (using e.g. suction or a negative pressure at the ports 231 of the first end of the catheter 200).

The catheter 200 in fig. 2 further comprises an inflatable retention element 250, such as an inflatable balloon, fixated to the elongated body 210 and at least partially surrounding the elongated body 210. The inflatable retention element 250 may be of different sizes depending on the intended use of the catheter 200 and similarly the inflatable retention element 250 may be made of any suitable flexible or elastic material, such as a plastic material. The inflatable retention element 250 forms an inflatable volume 251 which is in fluid communication with the inflation lumen 220 via a second inflation port 222. The inflation lumen 220 extends to a first inflation port 221 on the first end of the catheter 200. By injecting a fluid into the inflation lumen 220 via the first inflation port 221 the inflatable volume 251 may be increased and by e.g. providing a suction or venting of the inflation lumen 220 the volume 251 of the inflatable retention element 250 may be decreased. Accordingly, by inserting the elongated body 210 into e.g. the anal canal or rectum of the patient, with the inflatable retention element 250 in a deflated state, the inflatable retention element 250 may be inflated by injecting a fluid into the inflation lumen 220, such as water or air, whereby the inflatable retention element 250 inflates to retain the catheter in the inserted position, and may additionally form a seal against the body of the patient for retaining fluids inside the patient, such as fluids injected via the second port 232 of the main lumen 230 inside the patient.

It is further noted that while the main lumen(s) 230 and the inflation lumen 220 are depicted as separate lumens being arranged side-by-side in the catheter 200 other channel layouts are possible, e.g. the main lumen 230 may be concentrically placed inside inflation lumen 220 or vice versa.

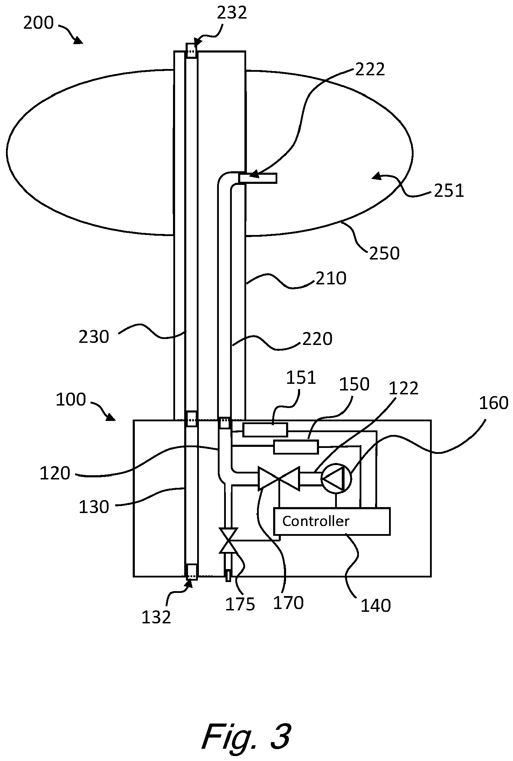

Fig. 3 illustrates the catheter control assembly 100 being connected to the catheter 200. It is noted that while the catheter control assembly 100 is depicted as being directly connected to the catheter 200 the catheter control assembly 100 may be connected to the corresponding lumens of the catheter 200 via one or more extension tubes (not shown).

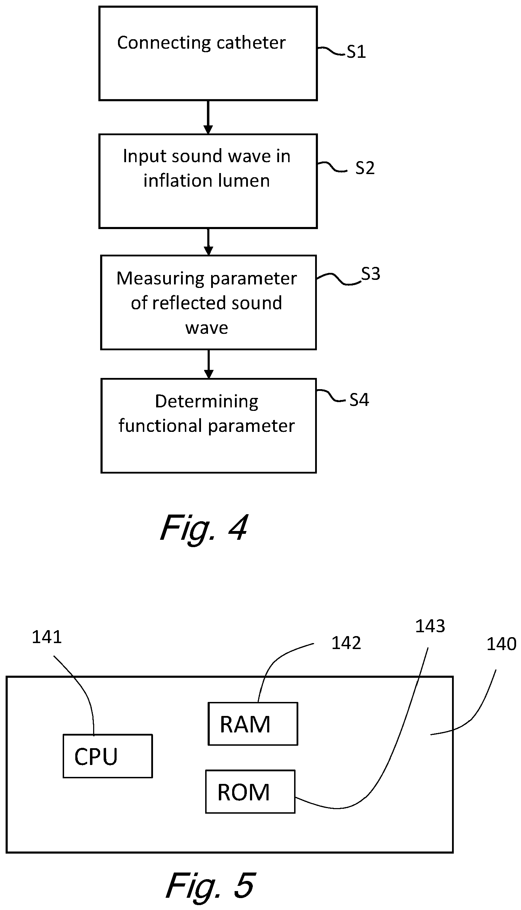

With further reference to Fig. 4 a method for determining a functional parameter of the catheter 200 will now be described.

At step S1 a catheter 200 is connected to a catheter connector interface comprising a second lumen 130 configured to be in fluid communication with a main lumen 230 of the catheter 200 and a first lumen 120 configured to be in fluid communication with an inflatable retention element 250 of the catheter 200. If the catheter 200 does not comprise an inflatable retention element 250 the first lumen 120 of the catheter connector interface configured to be in fluid communication with an inflatable retention element 250 of the catheter 200 may be in fluid connection with a lumen of the catheter 200 which is closed, i.e. is not in fluid communication with an inflatable retention element 250 or the environment of the catheter 200. As a further example, the catheter 200 may lack the inflation lumen 220 altogether whereby the first lumen 120 of the catheter arrangement 100 is closed at the inflation port 121 when the catheter 200 is connected to the catheter connector interface. Alternatively, the second inflation port 222 may be closed meaning that the second lumen 120 is in fluid communication with the substantially fixed volume of the inflation lumen 220.

Some catheters used for rectal irrigation does not comprise an inflatable retention element which is in fluid communication with the first lumen 220 and instead comprises no retention elements at all, or other types of retention elements, such as a cone element pointing towards the second end of the catheter 200 (comprising the second port 232) and provided around the elongated body 210. The cone element is used as an alternative method for retaining the catheter in place and creating a seal against the patient's body. As an alternative to a cone element the catheter may comprise a closed (or static) fluid filled volume (e.g. a ball shaped element made from a non-elastic material) which is at least partially filled with a fluid when the user inserts the catheter, uses the catheter for irrigation and removes the catheter. While this closed fluid filled volume in some sense is an inflatable retention element it is not intended to be inflated after the user has inserted the catheter and it would not be in fluid communication with the first lumen 220. Accordingly, the detected parameter will indicate that the first lumen 220 is not in fluid communication with an inflatable retention element when a closed (or static) fluid filled volume is attached to the catheter to facilitate proper sealing during use as the closed (or static) fluid filled volume is isolated from the first lumen 220.

It is noted that some catheters may comprise an inflatable retention element 250, a cone element or both.

At step S2 a sound wave is input into the first lumen 120 of the catheter arrangement 100, by operation of the speaker 150, and the sound wave will propagate also into the inflation lumen 220 (if present in the catheter 200) and into the inflatable retention element 250. The sound wave will then, so some extent, be reflected back as an echo, and propagate back to the sensor 151.

The method proceeds to step S3 comprising measuring the pressure parameter. Step S3 may comprise measuring of at least one parameter related to the reflected sound wave with the sensor 151. The measurement(s) of the parameter acquired by the sensor 151 may be provided to a processing unit, such as the controller 140, configured to determine, based on the measurement(s), a functional parameter of the catheter 200. Accordingly, the method may go to step S4 comprising determining a functional parameter of the catheter 200 based on the measured pressure parameter.

The controller 140 can be realized by e.g. a central processing unit, CPU, 141, connected to memory or storage units, such as a RAM memory 142 and/or a ROM memory 143.

The sound wave input into the first lumen may preferably be in the form of a sound wave having a predetermined limited duration, and preferably in the form of a sound impulse/pulse. Such sound impulses/pulses may be very distinct, and easy to measure on. The predetermined limited duration may be between 1 millisecond and 1 second and preferably between 1 millisecond and 0.5 seconds and more preferably between 1-10 milliseconds. However, shorter impulses may also be used, such as in the range of 0.01-1 milliseconds, and preferably in the range of 0.1-0.5 milliseconds.

The sound wave is preferably generated by a sound generator, such as by a speaker. However, alternatively it is also possible to use other sound generating devices, and for example other parts of the assembly generating sounds that will spread into the first lumen. For example, the sound may be generated by a pump, such as the pump for inflation of the inflatable retention member or the pump for pumping liquid through the second lumen, such as irrigation liquid.

The generated and inputted sound may be of a single frequency, and preferably by a predetermined frequency. However, the sound may also have multiple frequencies, such as two simultaneously generated and inputted frequencies, or more than two frequencies. In an embodiment, the sound may e.g. comprise a chord having three or more frequencies.

The controller is preferably arranged to determine one or several parameters related to the reflected sound wave, based on the sensor data received from the sensor/microphone 151. The parameters to be determined by the controller 140 may e.g. be at least one of, and preferably two, and more preferably all three of:
- Response time, i.e. the time it takes between input of the sound wave into the first lumen and the initial detection of the reflected sound wave.
- The frequency of the returning sound wave, and in particular in comparison with the frequency of the input sound wave.
- The power/amplitude of the returning sound wave, and in particular in comparison with the power/amplitude of the input sound wave.

Preferably, the controller is further arranged to measure and determine the same parameters also for the input sound wave, i.e. to detect when the input sound wave is initiated, the frequency of the input sound wave and/or the amplitude of the input sound wave.

Based on the measured parameters related to the reflected sound wave, the controller can determine one or several functional parameters of the catheter. In particular, the controller may determine one, and preferably several, of the following functional parameters:
- The size of the catheter. The catheter may e.g. be available in two or three different sizes, such as small, regular and large. The different sizes may e.g. have different lengths and/or different diameters.
- The size of the balloon, i.e. the inflatable retention element. The size may e.g. depend on the degree of inflation.
- Whether the balloon, i.e. the inflatable retention element, has been used before or not. In particular, it may be determined whether the balloon has previously been fully inflated or not. The balloon may be partly inflated as part of a priming and initialization process. However, such a limited inflation does generally not affect the properties of the balloon, and would not be recognizable as a previous use. However, a full inflation, as in ordinary use, will affect the properties of the balloon, and may make the balloon unsuitable for repeated use. Such a previous use may be detected by the controller.
- In embodiments, it may also, or alternatively, the status or condition of the balloon may be determined, which may be dependent on how many times the catheter has been used (for a reusable catheter). Hereby, it may be determined that the catheter and balloon have reached a point where it is unsuitable for further use.
- It may further be determined whether there is a catheter connected to the control assembly or not.
- It may also be determined whether the catheter has an inflatable retention element (balloon) or not. For example, the catheter may instead have a non-inflatable retention element, or no retention element at all.
- It may also be determined whether the catheter has an inflation lumen or not. For example catheter having non-inflatable retention elements may still have an inflation lumen, but would normally not have such a lumen.
- The controller may also determine whether the catheter is in an operative position, such as inserted in the rectum in case of a rectal catheter, or inserted in the urethra for a urinary catheter, or positioned outside the body.

For making the determination, the controller 140 may comprise an artificial neural network. The neural network may be a feedforward network or a recurrent network. The neural network may be trained, e.g. in a conventional computer. For training, catheters with known functional parameters, such as being known to be new or previously used/inflated, known to be of a particular size, such as small, regular or large, known to be inside or outside the body, etc, may be tested, and the one or more parameters related to the reflected sound wave may be measured for each sample catheter. Thus, a number of samples are preferably tested for each combination, i.e. for each permutation, of functional features that are to be determinable by the controller. Preferably, many samples for each combination/permutation are used, and in most cases the more samples that are used for the training, the more exact the determination will be.

For example, if two available catheter sizes are available (short and long), the following catheter configurations could be evaluated:
- Short catheter - not used
- Short catheter - used with few inflations
- Short catheter -used with many inflations
- Long catheter - not used
- Long catheter - used with few inflations
- Long catheter -used with many inflations
- No catheter connected
- Catheter with non-inflatable retention element connected

Further, the conditions of various inflation levels may be evaluated, such as an inflated balloon diameter of 20 mm, 30 mm, 40 mm, 50 mm, 60 mm and 70 mm. However, other inflation diameters may be considered instead.

A number of samples for each test situation may be measured, such as 1, 10, 100 or 1000 for each situation.

Alternatively, one or more of the functional parameters may be determined by comparing a measured parameter for the reflected sound wave with a database of previously measured parameter samples obtained for catheters with different functional parameters and determining the closest matching parameter samples. The associated functional parameter of the closest matching sequence may be the determined functional parameter. It is noted that many alternatives exist for determining the functional parameter based on parameter(s) of the reflected sound wave given reference measurements of the parameter for characters with known functional parameters.

It is also feasible to establish a direct correlation between one or more of the function parameters with one or more of the measured parameters related to the reflected sound wave.

The determined functional parameter of the catheter may be used in various ways. For example, the controller may be arranged to issue an alarm upon determination of a certain functional parameter, such as the non-presence of a catheter connected to the interface, the presence of an already used catheter, etc. The alarm may e.g. be issued as a text message, activation of a light, activation of a sound alarm, or the like.

Additionally, or alternatively, the controller may be arranged to control the operation of the catheterization procedure, such as by automatically activating inflation of the inflatable retention element when an inflatable retention member has been detected, automatically controlling the inflation to a suitable inflation level, dependent on the size of the balloon or catheter, automatically deactivating inflation of the inflatable retention element, e.g. when no catheter has been detected, or when the catheter is determined not to have an inflatable retention element, automatically aborting an already activated inflation procedure, e.g. when a maximum inflated size has been reached, etc.

The person skilled in the art realizes that the present invention by no means is limited to the preferred embodiments described above. On the contrary, many modifications and variations are possible within the scope of the appended claims. For example, the sensor may measure also additional parameters related to the reflected soundwave. Further, the controller for determining the functional parameter may be a controller dedicated only for this purpose, or may be arranged as a part of a controller controlling the operation of the assembly, such as an irrigation controller. Such and other modifications should be construed to be within the scope of the present invention, as defined by the appended claims.

## Claims

1. A method for determining a functional parameter of a catheter, the method comprising:
a) connecting said catheter to a catheter connector interface, said catheter connector interface comprising a first lumen configured to be in fluid communication with an inflatable retention element of the catheter and a second lumen configured to be in fluid communication with a main lumen of the catheter;
b) inputting a sound wave into the first lumen;
c) measuring at least one parameter of a reflection of the sound wave in the first lumen; and
d) determining, based on the measured parameter, a functional parameter of the catheter.

2. The method according to claim 1, wherein the measuring of the at least one parameter of the reflection of the sound wave in the first lumen comprises measuring of the time it takes for the reflection of the sound wave to be detectable after inputting of the sound wave into the first lumen.

3. The method according to claim 1 or 2, wherein the measuring of the at least one parameter of the reflection of the sound wave in the first lumen comprises measuring of the frequency or frequencies of the reflected sound wave.

4. The method according to any one of the claims 1-3, wherein the measuring of the at least one parameter of the reflection of the sound wave in the first lumen comprises measuring of the amplitude of the reflected sound wave.

5. The method according to any one of the preceding claims, wherein the measuring of the at least one parameter of the reflection of the sound wave in the first lumen comprises measuring of the phase of the reflected sound wave.

6. The method according to any one of the preceding claims, wherein the determined functional parameter indicates either that the catheter comprises a new inflatable retention element or a used inflatable retention element.

7. The method according to any one of the preceding claims, wherein the sound wave is input into the first lumen in the form of a sound wave having a predetermined limited duration, and preferably in the form of a sound impulse or pulse.

8. The method according to claim 7, wherein said predetermined limited duration is between 1 millisecond and 1 second and preferably between 1 millisecond and 0.5 seconds and more preferably between 1-10 milliseconds.

9. The method according to any one of the preceding claims, wherein the functional parameter is at least one of: the size of the inflatable retention element, the status or condition of the inflatable retention element, and the size of the catheter.

10. The method according to any one of the preceding claims, wherein the functional parameter is at least one of: the presence of a catheter and the presence of a fluid communication with the inflatable retention element.

11. The method according to any of the preceding claims, wherein said functional parameter is indicative of at least one of: whether the catheter comprises an inflatable retention element, whether the inflatable retention element is new or used, and a size of the inflatable retention element.

12. The method according to any one of the preceding claims, further comprising continuous or regular repetition of the steps b)-d).

13. A catheter control assembly comprising:
a catheter connector interface, comprising:
a first lumen configured to be in fluid communication with an inflatable retention element of the catheter;
a second lumen configured to be in fluid communication with a main lumen of a catheter;
a sound generator arranged to input a sound wave into the first lumen; and
a catheter controlling unit, the catheter controlling unit being configured to
measure at least one parameter of a reflection of the sound wave in the first lumen; and
determine, based on the measured pressure parameter, a functional parameter of the catheter.

14. The catheter control assembly of claim 13, further comprising a microphone connected to said catheter controlling unit for measuring of the at least one parameter of the reflected sound wave.

15. The catheter control assembly of claim 13 or 14, wherein the sound generator is a speaker.

16. A computer program product comprising instructions which, when executed by a computer, performs the method according to any of claims 1-12.
